# EUROPÄISCHE PATENTSCHRIFT

(11) **EP 1 231 982 B1**
(45) Veröffentlichungstag und Bekanntmachung des Hinweises auf die Patenterteilung: **21.03.2007**
(21) Anmeldenummer: 00934980.4
(22) Anmeldetag: 03.05.2000
(51) Int. Cl.: A61P 9/00, A61P 25/00, A61K 31/495, A61K 31/502

(54) **VERWENDUNG VON PHTHALAZIN-DERIVATEN ZUR BEHANDLUNG NEURODEGENERATIVER KRANKHEITEN**
USE OF PHTHALAZINE DERIVATIVES FOR THE TREATMENT OF NEURODEGENERATIVE DISEASES
UTILISATION DE DERIVES DE PHTALAZINE POUR LE TRAITEMENT DES MALADIES NEURODEGENERATIVES

(30) Priorität: 11.05.1999 DE 19921567
(43) Veröffentlichungstag der Anmeldung: 21.08.2002
(73) Patentinhaber: Abbott GmbH & Co. KG, 65205 Wiesbaden (DE)
(72) Erfinder: LUBISCH, Wilfried, D-69115 Heidelberg (DE); SADOWSKI, Jens, D-67117 Limburgerhof (DE); KOCK, Michael, D-67105 Schifferstadt (DE); HÖGER, Thomas, D-68535 Edingen-Neckarhausen (DE)
(74) Vertreter: Grünecker, Kinkeldey, Stockmair & Schwanhäusser Anwaltssozietät
(86) Internationale Anmeldenummer: PCT/EP2000/003967
(87) Internationale Veröffentlichungsnummer: WO 2000/067734

(56) Entgegenhaltungen:
- EP-A- 0 722 936
- WO-A-00/05218
- WO-A-00/05219
- WO-A-98/45292
- WO-A-99/11649
- GB-A- 2 112 389
- US-A- 4 939 140
- BERNARD, A. M. ET AL: "A new and efficient synthesis of phthalazin-1(2H)-ones" SYNTHESIS (1998), (3), 317-320 , XP001010445
- MIYAZAKI, H. ET AL: "The use of stable isotopes in drug metabolism and pharmacokinetics" ADV. PHARMACOL. THER., PROC. INT. CONGR., 8TH (1982), MEETING DATE 1981, VOLUME 6, 227-33. EDITOR(S): YOSHIDA, HIROSHI: HAGIHARA, YASHIREO;EBASHI, SETSURO. PUBLISHER: PERGAMON, OXFORD, UK. , XP001014487

## Beschreibung

Die vorliegende Erfindung betrifft die Verwendung von Phthalazin-Derivaten als Inhibitoren des Enzyms Poly(ADP-ribose)polymerase oder PARP (EC 2.4.2.30), die Verwendung als Inhibitoren von PARP-homologen Enzymen und insbesondere zeigen diese Phthalazin-Derivate auch eine selektive Hemmung der PARP-homologen Enzyme.

Poly(ADP-ribose)polymerase (PARP) bzw. wie es auch genannt wird Poly(ADP-ribose)synthase (PARS) stellt ein regulatorisches Enzym dar, das in Zellkernen gefunden wird (K. Ikai et al., *J. Histochem. Cytochem.* 1983, 31, 1261-1264). Man nimmt an, daß PARP eine Rolle bei der Reparatur von DNA-Brüchen spielt (M.S. Satoh et al., *Nature* 1992, 356, 356-358). Schädigungen oder Brüche der DNA-Stränge aktivieren das Enzym PARP, das, wenn es aktiviert ist, die Übertragung von ADP-Ribose aus NAD katalysiert (S. Shaw, *Adv. Radiat. Biol.,* 1984, 11, 1-69). Dabei wird Nikotinamid aus NAD freigesetzt. Nikotinamid wird unter Verbrauch des Energieträgers ATP von anderen Enzymen wieder in NAD umgewandelt. Eine Überaktivierung von PARP hätte dementsprechend einen unphysiologisch hohen Verbrauch von ATP zur Folge und dies führt im Extremfall zu Zellschädigungen und Zelltod.

Es ist bekannt, daß Radikale wie Superoxid-Anion, NO und Wasserstoffperoxid in Zellen zu DNA-Schädigungen führen können und damit PARP aktivieren. Die Bildung von großen Mengen an Radikalen wird bei einer Reihe von pathophysiologischen Zuständen beobachtet und man geht davon aus, daß diese Anhäufung von Radikalen zu den beobachteten Zell- bzw Organschäden führen oder beitragen. Dazu zählt von zum Beispiel ischämische Zustände von Organen wie im Schlaganfall, Herzinfarkt (C. Thiemermann et al., *Proc. Natl. Acad. Sci. USA,* 1997, 94, 679-683) oder Ischämie der Nieren, aber auch Reperfusionsschäden wie sie zum Beispiel nach der Lyse von Herzinfarkt auftreten (s. oben: C. Thiemermann et al.). Die Hemmung von dem Enzym PARP könnte demzufolge ein Mittel sein, um diese Schäden zum mindestens zum Teil zu verhindern oder abzumildern. PARP-Inhibitoren könnten somit ein neues Therapieprinzip zur Behandlung von eine Reihe von Krankheiten darstellen.

Das Enzym PARP beeinflußt die Reparatur von DNA-Schäden und könnte somit auch in der Therapie von Krebs-Erkrankungen eine Rolle spielen, da in Kombination mit cytostatisch wirksamen Stoffen ein höheres Wirkpotential gegenüber Tumorgewebe beobachtet wurde (G. Chen et al. *Cancer Chemo. Pharmacol.* 1988, *22,* 303).

Zudem wurde gefunden, daß PARP-Inhibitoren immunosuppressive Wirkung zeigen können (D. Weltin et al. *Int. J. Immunopharmacol.* 1995, 17, 265-271).

Es wurde ebenfalls entdeckt, daß PARP bei immunologischen Erkrankungen bzw. Krankeiten, in denen das Immunsystem eine wichtige Rolle spielt, wie zum Beispiel rheumatoide Arthritis und septischer Schock, involviert ist, und daß PARP-Inhibitoren einen günstigen Effekt auf den Krankheitsverlauf zeigen können (H. Kröger et al. *Infammation* 1996, *20,* 203-215; W. Ehrlich et al. *Rheumatol. Int.* 1995, 15, 171-172; C. Szabo et al., *Proc. Natl. Acad. Sci. USA* 1998, 95, 3867-3872; S. Cuzzocrea et al. *Eur. J. Pharmacol.* 1998, *342,* 67-76).

Weiterhin zeigte der PARP-Inhibitor 3-Aminobenzamid protektive Effekte in einem Model für den Kreislaufschock (S. Cuzzocrea et al., *Br. J. Pharmacol.* 1997, *121,* 1065-1074).

Ebenfalls gibt es experimentelle Hinweise, das Inhibitoren des Enzymes PARP als Mittel zur Behandlung von Diabetes mellitus nützlich sein könnten (V. Burkart et al. *Nature Med.* 1999, 5, 314-319).

Phthalazine und deren Derivate stellen eine vielbenutzte Stoffklasse dar. 2H-Phthalazin-1-one, die zudem in 4-Stellung Substituenten tragen, sind jedoch bisher weniger beschrieben worden. So sind Methylenamide, Methylenharnstoffe und Methylenimide in Puodzhyunas et al. Pharm. Chem. J. 1973, 7, 566; W. Mazkanowa et al., Zh. Obshch. Khim. 1958, 28, 2822 und in F.K. Mohamed et al., Ind. J. Chem. B, 1994, 33, 769 dargestellt worden. Zyklische Amine und Alkylamine, wobei die Aminogruppe sowohl eine zyklische als auch ein aliphatisches Amin sein kann, sind in J. Singh et al., Ind. J. Chem. B, 1983, 22, 1083, Y. Egushi et al., Chem Pharm. Bull. 1991, 9, 1846 und in Iyo Kizai Kenkyusho Hokoku, 1998, 12, 41 (CA 91, 91579) beschrieben worden, wobei allerdings die Verbindungen auf antiatherosklerotische Wirkung, auf Hemmung der Blutplättchenaggragation oder auf Blutdrucksenkung untersucht wurden. In WO 99/11649 sind in Phthalazinone erwähnt, die in 4-Stellung Phenylpiperazinylmethyl-Reste tragen und die als Inhibitoren des Enzymes PARP beschrieben wurden.

In A.M. Bernard et al., Synthesis 1998, 317 sind 2H-Phthalazinone, in 4-Stellung Phenoxymethyl-Derivate tragen, hergestellt worden.

In der vorliegenden Erfindung werden neue Phthalazin-Derivate der allgemeinen Formeln I beschrieben, die überraschenderweise PARP-Inhibitoren darstellen.

Weiterhin wurde überraschend gefunden, daß diese Verbindungen der allgemeinen Formel I auch PARP-homologe Enzyme hemmen. Zudem zeigen diese Verbindungen überraschenderweise eine selektive Hemmung der PARP-homolgen Enzyme d.h. die Verbindungen hemmen die homologen Enzyme stärker als das Enzym PARP selbst.

Die erfindungsgemäßen Phthalazine weisen gegenüber PARP-Homologen (PARP 2) eine bevorzugt 5-fach stärkere inhibitorische Wirkung auf als gegenüber dem bekannten PARP (PARP 1).

Unter PARP-Homologen wird insbesondere das Homologe PARP-2 gemäß WO 99/64572 (human PARP2) verstanden. Dies läßt sich vorteilhafterweise aus dem menschlichen Hirn, Herz, skelettmuskel, Niere und Leber isolieren. In anderen Geweben oder Organen ist humanPARP2 deutlich schwächer exprimiert.

Insbesondere das aus menschlichem Hirn isolierbare humanPARP2 und dessen funktionale Äquivalente sind bevorzugte Agenzien für die Entwicklung von Inhibitoren gegen Schlaganfall. Es kann nämlich angenommen werden, daß die Wirkstoffentwicklung, basierend auf PARP2 als Indikator, die Entwicklung von Inhibitoren ermöglicht, welche für die Anwendung an menschlichem Gehirn optimiert sind. Es ist jedoch nicht auszuschließen, daß auf Basis von PARP2 entwickelte Inhibitoren auch zur Therapierung PARP-vermittelter pathologischer Zustände anderer Organe einsetzbar sind. Anhand der Gewebeverteilung der erfindungsgemäßen Proteine sind vor allem Indikationen von Interesse, die auf ischämischen Zuständen entsprechender Organe beruhen (Ischämie der Hirns (Schlaganfall), der Herzens (Herzinfarkt), Schädigungen, die während oder nach der Infarktlyse (z.B. mit TPA, Reteplase oder mechanisch mit Laser oder Rotoblator) und von Mikroinfarkten während und nach Herzklappenersatz, Aneurysmenresektionen und Herztransplantationen, der Niere (akuten Nierenversagen, akute Niereninsuffizienz oder Schädigungen während und nach einer Nierentransplantation), Schädigung der Leber oder der Skelettmuskulatur). Ferner sind Behandlung und Prophylaxe von neurodegenerativen Erkrankungen denkbar, die nach Ischämie, Trauma (Schädel-Hirn-Trauma), Massenblutung, Subarachnoidal-Blutungen und Schlaganfall auftreten, sowie von neurodegenerativen Erkrankungen, wie multipler Infarkt-Dementia, Alzheimer Krankheit, Huntington Krankheit und Epilepsien, insbesondere von generalisierten epileptischen Anfällen, wie z.B. Petit mal, und tonisch-clonischen Anfällen und partiell epileptischen Anfällen, wie Temporal Lope, und komplex-partiellen Anfällen. Ferner können besagte Proteine relevant sein bei der Behandlung einer Revaskularisation kritisch verengter Koronararterien und kritisch verengter peripherer Arterien, z.B. Beinarterien. Darüber hinaus können besagte Proteine eine Rolle spielen bei der Chemotherapie von Tumoren und bei der Verhinderung von Metastasierungen sowie bei der Behandlung von Entzündungen und rheumatischen Erkrankungen, z.B. der rheumatischen Arthritis. Weitere pathologische Zustände dieser und anderer Organe sind denkbar.

Ähnlich wie PARP1 wird auch PARP2 durch geschädigte DNA, wenn auch durch einen vermutlich anderen Mechanismus aktiviert. Eine Bedeutung in der DNA-Reparatur ist denkbar. Die Blockade der erfindungsgemäßen PARPs würde auch in Indikationen, wie Krebs, von Nutzen sein (z.B. in der Radiosensitisierung von Tumorpatienten).

Unter Verwendung der oben beschriebenen spezifischen Assay-Systeme für Bindungspartner von PARP1 und PARP2 wurden aktive und selektive Inhibitoren gegen die erfindungsgemäßen Proteine entwickelt.

Erfindungsgemäß bereitgestellte Inhibitoren besitzt gegenüber PARP2 eine stark ausgeprägte inhibitorische Aktivität. Die Kᵢ-Werte können dabei weniger als etwa 1000 nM, wie z.B. weniger als etwa 700 nM, weniger als etwa 100 nM und weniger als etwa 30 nM, wie z.B. etwa 1 bis 20 nM, betragen.

Erfindungsgemäß bevorzugte Inhibitoren besitzen eine überraschend ausgeprägte Selektivität für PARP2. Das Verhältnis Kᵢ(PARP1): Kᵢ(PARP2) für erfindungsgemäße Inhibitoren ist nämlich z.B. größer als 5. Eine weitere Gruppe von Inhibitoren wurde so entwickelt, daß sie PARP1 und PARP2 gleichzeitig inhibieren.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P.H. et al., Hrsg, Elsevier, Amsterdam-New York-Oxford, 1985) entnommen werden. Unter Vektoren sind außer Plasmiden auch alle anderen dem Fachmann bekannten Vektoren wie beispielsweise Phagen, Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

### Expression der Konstrukte

Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, beschrieben.

Als Wirtsorganismen sind prinzipiell alle Organismen geeignet, die eine Expression der erfindungsgemäßen Nukleinsäuren, ihrer Allelvarianten, ihrer funktionellen Äquivalente oder Derivate oder des rekombinanten Nukleinsäurekonstrukts ermöglichen. Unter Wirtsorganismen sind beispielsweise Bakterien, Pilze, Hefen, pflanzliche oder tierische Zellen zu verstehen. Bevorzugte Organismen sind Bakterien, wie solche der Gattungen Escherichia, wie z.B. Escherichia coli, Streptomyces, Bacillus oder Pseudomonas, eukaryotische Mikroorganismen, wie Saccharomyces cerevisiae, Aspergillus, höhere eukaryotische Zellen aus Tieren oder Pflanzen, beispielsweise Sf9 oder CHO-Zellen.

Gewünschtenfalls kann das Genprodukt auch in transgenen Organismen wie transgenen Tieren, wie insbesondere Mäusen, Schafen oder transgenen Pflanzen zur Expression gebracht werden. Bei den transgenen Organismen kann es sich auch um sogenannte Knock-Out Tiere oder Pflanzen handeln, in denen das korrespondierende endogene Gen ausgeschaltet wurde, wie z.B. durch Mutation oder partielle oder vollständige Deletion.

Die Kombination aus den Wirtsorganismen und den zu den Organismen passenden Vektoren, wie Plasmide, Viren oder Phagen, wie beispielsweise Plasmide mit dem RNA-Polymerase/Promoter System, die Phagen λ, µ oder andere temperente Phagen oder Transposons und/oder weiteren vorteilhaften regulatorischen Sequenzen bilden ein Expressionssystem. Bevorzugt sind unter dem Begriff Expressionssysteme beispielsweise die Kombination aus Säugetierzellen, wie CHO-Zellen, und Vektoren, wie pcDNA3neo-Vektor, die für Säugetierzellen geeignet sind, zu verstehen.

Wie oben beschrieben, kann das Genprodukt vorteilhaft auch in transgenen Tieren, z.B. Mäusen, Schafen oder transgenen Pflanzen zur Expression gebracht werden. Ebenso ist es möglich, zellfreie Translationssysteme mit der von der Nukleinsäure abgeleiteten RNA zu programmieren.

### Herstellung von Antikörpern:

Die Herstellung von Anti-PARP2-Antikörpern erfolgt in einer dem Fachmann geläufigen Weise. Mit Antikörpern sind sowohl polyklonale, monoklonale, humane oder humanisierte Antikörper oder Fragmente davon, single chain Antikörper oder auch synthetische Antikörper gemeint, ebenso wie Antikörperfragmente, wie Fv, Fab und (Fab)'₂. Geeignete Herstellungsverfahren sind z.B. beschrieben in Campbell, A.M., Monoclonal Antibody Technology, (1987) Elsevier Verlag, Amsterdam, New York, Oxford und in Breitling, F. und Dübel, S., Rekombinante Antikörper (1997), Spektrum Akademischer Verlag, Heidelberg.

### Beispiel A: Isolierung der PARP2-cDNA

Bei der Sequenzanalyse von cDNA-Klonen einer cDNA-Bibliothek aus menschlichem Gehirn (Human Brain 5'Stretch Plus cDNA Library, # HL3002a, Fa. Clontech) wurden die vorliegenden cDNA-Sequenzen erstmalig gefunden. Die Sequenz dieses Klons ist in SEQ ID NO:1 beschrieben.

### Beispiel B: Herstellung der Enzyme

Humanes PARP1 wurde zum Vergleich rekombinant im Baculovirus-System in der dem Fachmann geläufigen Weise exprimiert und wie beschrieben (Shah et al., Analytical Biochemistry 1995, 227, 1-13) partiell aufgereinigt. Rinder PARP1 in einer 30-50%igen Reinheit (c = 0,22 mg/ml, spez. Aktivität 170 nmol ADP-ribose/min/mg Gesamtprotein bei 25°C) wurde von BIOMOL (Best.-Nr. SE-165) bezogen. Humanes PARP2 wurden rekombinant im Baculovirus-System (Bac-to-Bac System, BRL LifeScience) exprimiert. Dazu wurden die entsprechenden cDNAs in den pFASTBAC-1-Vektor kloniert. Nach Herstellung von rekombinanter Baculovirus-DNA durch Rekombination in E. coli, erfolgte Transfektion von Insektenzellen (Sf9 oder High-Five) mit den entsprechenden rekombinanten Baculovirus-DNAs. Die Expression der entsprechenden Proteine wurde durch Western-Blot-Analyse verifiziert. Virenstämme wurden in der dem Fachmann geläufigen Weise amplifiziert. Größere Mengen rekombinanter Proteine wurden durch Infektion von 500 ml Insektenzellkultur (2 x 10⁶ Zellen/ml) mit Viren in einer MOI (multiplicity of infection; Verhältnis von Viren zu Zellen) von 5-10 infiziert und 3 bis 4 Tage inkubiert. Anschließend wurden die Insektenzellen durch Zentrifugation pelletiert und die Proteine aus dem Pellet aufgereinigt.

Die Aufreinigung erfolgte durch klassische, dem Fachmann geläufige Methoden der Proteinreinigung unter Detektion der Enzyme mit entsprechenden spezifischen Antikörpern. Teilweise wurden die Proteine auch über eine 3-Aminobenzamid/Affinitätssäule wie beschrieben (Burtscher et al., Anal Biochem 1986, 152:285-290) affinitätsgereinigt. Die Reinheit betrug >90%.

### Beispiel C: Testsysteme für die Bestimmung der von Aktivität von PARP2 und der Inhibitorischen Wirkung von Effektoren auf PARP1 und PARP2

### a) Herstellung von Antikörpern gegen Poly-(ADP-ribose)

Als Antigen zur Generierung von Anti-Poly-(ADP-ribose) Antikörpern kann Poly-(ADP-ribose) verwendet werden. Die Herstellung von Anti-Poly-(ADP-ribose) Antikörpern ist in der Literatur beschrieben. (Kanai Y et al. (1974) Biochem Biophys Res Comm 59:1, 300-306; Kawamaitsu H et al. (1984) Biochemistry 23, 3771-3777; Kanai Y et al. (1978) Immunology 34, 501-508).

Unter anderem wurden verwendet: Anti Poly-(ADP-ribose)-Antikörper (polyklonales Antiserum, Kaninchen), BIOMOL; Best.-Nr. SA-276. Anti Poly-(ADP-ribose)-Antikörper (monoklonal, Maus; Klon 10H; Hybri-omaüberstand, affinitätsgereinigt).

Die Antiseren oder aus Hybridomakulturüberstand gewonnenen monoklonalen Antikörper wurden durch eine Protein-A-Affinitätschromatographie in der dem Fachmann geläufigen Weise aufgereinigt.

### b) ELISA-Assay

### Materialien:

### ELISA Farbreagenz: TMB-Fertigmix SIGMA T-8540

Eine 96-well Mikrotiterplatte (FALCON Micro-Test IIIä Flexible Assay Plate, # 3912) wurde mit Histonen (SIGMA, H-7755) beschichtet. Histone wurden hierfür in Carbonatpuffer (0.05M Na₂HCO₃; pH 9.4) in einer Konzentration von 50 µg/ml gelöst. Die einzelnen Wells der Mikrotiterplatte wurden mindestens 2 Stunden bei Raumtemperatur oder über Nacht bei 4°C mit je 150 µl dieser Histon-Lösung inkubiert. Anschließend werden die Wells durch Zugabe von 150 µl einer 1%igen BSA-Lösung (SIGMA, A-7888) in Carbonatpuffer für 2 Stunden bei Raumtemperatur blockiert. Es folgen drei Waschschritte mit Waschpuffer (0,0 5% Tween10 in 1x PBS; PBS (Phosphate buffered saline; Gibco, Best.-Nr. 10010): 0,21 g/l KH₂PO₄, 9 g/l NaCl, 0,726 g/l Na₂HPO₄ · 7H₂O, pH 7,4). Waschschritte wurden durchweg mit einem Mikrotiterplatten-Waschgerät durchgeführt (Mikrotiterplatten-Wäscher "Columbus", SLT-Labinstruments, Österreich).

Für die Enzymreaktion wurden eine Enzymreaktionslösung und eine Substratlösung jeweils als "Pre-Mix" benötigt. Die absolute Menge dieser Lösungen richtete sich nach der Anzahl der vorgesehenen Test-Wells.

Zusammensetzung der Enzymreaktionslösung pro Well:
- 4 µl PARP-Reaktionspuffer (1M Tris-HC1 pH 8,0, 100 mM MgCl₂, 10 mM DTT)
- 20 ng PARP1 (human oder bovin) oder 8 ng PARP2 (Human)
- 4 µl aktivierte DNA (1 mg/ml; SIGMA, D-4522)
- ad 40 µl H₂O

Zusammensetzung der Substrat-Lösung pro Well:
- 5 µl PARP-Reaktionspuffer (10x)
- 0,8 µl NAD-Lösung (10 mM, SIGMA N-1511)
- 44 µl H₂O

Inhibitoren wurden in 1x PARP-Reaktionspuffer gelöst. DMSO, daß gelegentlich zum Lösen von Inhibitoren in höheren Konzentrationen verwendet wurde, war bis zu einer Endkonzentration von 2 % unproblematisch. Für die Enzymreaktion wurden 40 µl der Enzymreaktionslösung pro Well vorgelegt und mit 10 µl Inhibitor-Lösung für 10 Minuten inkubiert. Anschließend wurde die Enzymreaktion durch Zugabe von 50 µl Substrat-Lösung pro Well gestartet. Die Reaktion wurde 30 Minuten bei Raumtemperatur durchgeführt und anschließend durch dreimaliges Waschen mit Waschpuffer gestoppt.

Als primäre Antikörper wurden spezifische Anti-Poly-(ADP-ribose) Antikörper in einer 1:5000 Verdünnung eingesetzt. Die Verdünnung erfolgte in Antikörper-Puffer (1 % BSA in PBS; 0,05 % Tween20). Die Inkubationszeit für den primären Antikörper betrug eine Stunde bei Raumtemperatur. Nach anschließendem dreimaligem Waschen mit Waschpuffer erfolgte eine einstündige Inkubation bei Raumtemperatur mit dem sekundärem Antikörper (Anti-Maus-IgG, Fab-Fragmente, Peroxidase gekoppelt, Boehringer Mannheim, Best.-Nr. 1500.686; Anti-Rabbit-IgG, Peroxidase gekoppelt, SIGMA, Best.-Nr. A-6154) in einer 1:10000 Verdünnung in Antikörper-puffer. Nach dreimaligem Waschen mit Waschpuffer folgte die Farbreaktion unter Verwendung von 100 µl Farbreagenz (TMB-Fertigmix, SIGMA) pro Well für ca. 15 min. bei Raumtemperatur. Die Farbreaktion wurde durch Zugabe von 100 µl 2M H₂SO₄ gestoppt. Danach wurde sofort im ELISA-Platten-Lesegerät ("Easy Reader" EAR340AT, SLT-Labinstruments, Österreich) gemessen (450nm gegen 620nm). Das Messprinzip ist schematisch in Figur 6 dargestellt.

Für die Ermittlung des Kᵢ-Wertes eines Inhibitors wurden verschie-dene Konzentrationen zur Erstellung einer Dosis-Wirkungskurve herangezogen. Für eine bestimmte Inhibitorkonzentration werden 3fach-Werte erhoben. Arithmetische Mittelwerte werden mit Microsoft^{©} Excel ermittelt. Die IC₅₀-Bestimmung erfolgt mit der Microcal^{©} Origin Software (Vers. 5.0) ("Sigmoidal Fit"). Umrechnung der so berechneten IC₅₀-Werte auf Kᵢ-Werte erfolgte durch Verwendung von "Eich-Inhibitoren". Die "Eich-Inhibitoren" wurden bei jeder Analyse mitgemessen. Der Kᵢ-Werte der "Eich-Inhibitoren" wurde im gleichen Testsystem durch Dixon-Diagramm Analyse in der dem Fachmann geläufigen Weise ermittelt.

### c) HTRF-(Homogenous time-resolved fluorescence) Assay

Beim erfindungsgemäßen HTFR-PARP-Assay werden Histone als Zielproteine der Modifikation durch PARP indirekt mit einem XL665-Fluorophor markiert. Der Antikörper wird direkt mit einem Europium-Kryptat markiert. Befindet sich das XL665-Fluorophor in einer unmittelbaren räumlichen Nähe, die durch eine Bindung an die Poly-(ADP-ribose) am Histon gewährleistet wird, dann ist eine Energieübertragung möglich. Die Emission bei 665 nm ist somit direkt proportional zu der Menge an gebundenem Antikörper, der wiederum der Poly-(ADP-ribose) Menge entspricht. Somit entspricht das gemessene Signal der PARP Aktivität. Das Meßprinzip ist schematisch in Figur 7 dargestellt. Die verwendeten Materialien sind, wenn nicht ausdrücklich angegeben, identisch mit denen im ELISA Assay (s.o.) verwendeten.

Histone wurden in Hepes-Puffer (50 mM, pH = 7,5) zu 3 mg/ml gelöst. Biotinylierung erfolgte mit Sulfo-NHS-LC-Biotin (Pierce, #21335T). Ein molares Verhältnis von 4 Biotin pro Histon wurde verwendet. Die Inkubationszeit betrug 90 Minuten (RT). Anschließend wurden die biotinylierten Histone über eine G25 SF HR10/10 Säule (Pharmacia, 17-0591-01) in Hepes Puffer (50 mM, pH = 7,0) aufgereinigt, um überschüssiges Biotinylierungsreagenz zu entfernen. Der Anti-Poly-(ADP-ribose)-Antikörper wurde mittels bifunktionaler Kopplungsreagenzien mit Europium-Kryptat markiert (Lopez, E. et al., Clin. Chem. 39(2), 196-201 (1993); US-Patent 5,534,622). Die Reinigung erfolgte auf einer G25SF HR10/30 Säule. Ein molares Verhältnis von 3.1 Kryptaten pro Antikörper wurde erzielt. Die Ausbeute betrug 25 %. Die Konjugate wurden in Gegenwart von 0,1 % BSA in Phosphatpuffer (0,1 M, pH = 7) bei -80°C gelagert.

Für die Enzymreaktion wurden pro Well zusammenpipettiert:
- 10 µl PARP-Lösung in PARP-HTRF-Reaktionspuffer (50 mM Tris-HCl pH 8,0, 10 mM MgC12, 1 mM DTT) mit 20 ng PARP1 (human oder bovin) oder 8ng PARP2 (Human)
- 10 µl aktivierte DNA in PARP-HTRF-Reaktionspuffer (50 µg/ml)
- 10 µl biotinylierte Histone in PARP-HTRF-Reaktionspuffer (1,25 µM)
- 10 µl Inhibitor in PARP-HTRF-Reaktionspuffer

Diese Reagenzien wurden 2 Minuten vorinkubiert, bevor die Reaktion durch Zugabe von
- 10 µl NAD-Lösung in PARP-HTRF-Reaktionspuffer (400 µM/ml) gestartet wurde. Die Reaktionszeit betrug 30 Minuten bei Raumtemperatur.

Anschließend wurde die Reaktion durch Zugabe von
- 10 µl PARP-Inhibitor (25 µM, Kᵢ=10nM) in "Revelation"-Puffer (100 mM Tris-HC1 pH 7,2, 0,2M KF, 0,05 % BSA)
gestoppt.

Danach wurden zugegeben:
- 10 µl EDTA-Lösung (SIGMA, E-7889, 0,5M in H₂O)
- 100 µl Sa-XL665 (Packard Instruments) in "Revelation"-Puffer (15-31,25nM)
- 50 µl Anti-PARP-Kryptat in "Revelation"-Puffer (1,6-3,3nM).

Nach 30 Minuten (bis 4 Stunden) konnte dann gemessen werden. Die Messung erfolgte auf einem "Discovery HTRF Microplate Analyzer" (Packard Instruments). Die Berechnung der Kᵢ-Werte erfolgte wie beim ELISA Assay beschrieben.

Gegenstand der vorliegenden Erfindung ist die Verwendung von substituierten Phthalazine der allgemeinen Formel I 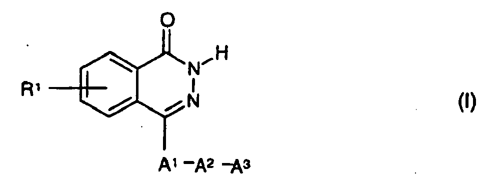 worin
- R¹: Wasserstoff, Chlor, Fluor, Brom, Jod, verzweigtes und unverzweigtes C₁-C₆-Alkyl, OH, Nitro, CF₃, CN, NR¹¹R¹², NH-CO-R¹³, O-C₁-C₄-Alkyl, wobei R¹¹ und R¹² unabhängig voneinander Wasserstoff oder C₁-C₄-Alkyl bedeuten und R¹³ Wasserstoff, C₁-C₄-Alkyl, C₁-C₄-Alkyl-Phenyl oder Phenyl bedeuten, und
- A¹: einen geradkettigen oder verzweigten C₀-C₆-Alkylrest und
- A²: NR², NR²-C₁-C₆-Alkyl-, und S und
- R²: Wasserstoff und C₁-C₆-Alkyl und
- A³: einen aromatischen oder heteroaromatischen ein oder zweigliedrigen Ring mit je 5 oder 6 Ringatomen und bis zu 3 Heteroatomen, ausgewählt aus N, O, S, wie zum Beispiel Phenyl, Thiophen, Pyridin, Pyrimidin, Naphthalin, Indol, Imidazol, die noch mit R⁴ und einem oder zwei R³ substituiert sein können wobei R³ Wasserstoff, Chlor, Fluor, Brom, Jod, verzweigtes und unverzweigtes C₁-C₆-Alkyl, OH, Nitro, CF₃, CN, NR¹¹R¹², SO₂NR¹¹R¹², SO₂-C₁-C₄-Alkyl, S-C₁-C₄-Alkyl, O-Ph, O-CF₃, NH-CO-R¹³, O-C₁-C₄-Alkyl, wobei R¹¹ und R¹² unabhängig voneinander Wasserstoff oder C₁-C₄-Alkyl bedeuten und R¹³ Wasserstoff, C₁-C₄-Alkyl, C₁-C₄-Alkyl-Phenyl oder Phenyl bedeuten kann,
- R⁴: Wasserstoff, (X)_{0,1}-C₁-C₄-Alkyl-NR⁴¹R⁴², wobei X = O, S und NR⁴³ und R⁴¹ und R⁴² unabhängig voneinander Wasserstoff, C₁-C₆-Alkyl, C₁-C₄-Alkyl-Phenyl und ein cyclisches Amin von 3 bis 7 Gliedern sein kann und R⁴³ Wasserstoff und C₁-C₄-Alkyl sein kann,
sowie ihre tautomeren Formen, möglichen enantiomeren und diastereomeren Formen, und deren Prodrugs.

Die Verbindungen der Formel I können als Racemate, als enantiomerenreine Verbindungen oder als Diastereomere eingesetzt werden. Werden enantiomerereine Verbindungen gewünscht, kann man diese beispielweise dadurch erhalten, daß man mit einer geeigneten optisch aktiven Base oder Säure eine klassische Racematspaltung mit den Verbindungen der Formel I oder ihren Zwischenprodukten durchführt.

Gegenstand der Erfindung sind auch zu Verbindungen der Formel I mesomere oder tautomere Verbindungen.

Ein weiterer Gegenstand der Erfindung sind die physiologisch verträglichen Salze der Verbindungen I, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd. 10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid, Kaliumhydroxid und Tris.

Unter Prodrugs werden solche Verbindungen verstanden, die *in vivo* in Verbindungen der allgemeinen Formel I metrabolisiert werden. Typische Prodrugs sind Phosphate, Carbamate von Aminosäuren, Ester und andere.

Die Herstellung der erfindungsgemäßen Phthalazin-Derivate I kann auf verschiedenen Wegen, die in der Literatur bereits durchgeührt wurden, erfolgen.

Die möglichen Synthesemethoden sind zum Beispiel in Puodzhyunas et al. Pharm. Chem. J. 1973, 7, 566, W. Mazkanowa et al., Zh. Obshch. Khim. 1958, 28, 2822, F.K. Mohamed et al., Ind. J. Chem. B, 1994, 33, 769, J. Singh et al., Ind. J. Chem. B, 1983, 22, 1083, Y. Egushi et al., Chem Pharm. Bull. 1991, 9, 1846 und in Iyo Kizai Kenkyusho Hokoku, 1998, 12, 41 (CA 91, 91579) beschrieben oder dort zitiert worden. Die erfindungsgemäßen Verbindungen können analog der dort beschriebenen Methoden hergestellt werden.

Die in der vorliegenden Erfindung enthaltenen substituierten Phthalazine I stellen Inhibitoren des Enzyms Poly(ADP-ribose)-polymerase dar und zeigen insbesondere Selektivität für neue PARP-homolge Enzyme.

Die inhibitorische Wirkung der substituierten Phthalazin-Derivate I wurde mit einem in der Literatur bereits bekannten Enzymtest ermittelt, wobei als Wirkmaßstab ein Ki-Wert ermittelt wurde. Die Phthalazin-Derivate I wurden in dieser Weise auf Hemmwirkung des Enzyms Poly(ADP-ribose)polymerase oder PARP (EC 2.4.2.30) gemessen.

Das 4-(4-Phenylpiperazin-1-yl)methyl-2H-phthalazin-1-on wurde in WO 99/11649 als PARP-Inhibitor beschrieben und ist den erfindungsgemäßen Verbindungen strukturell verwandt. Diese Verbindung wurde im angegebenen HTRF-Assay auf die Hemmwirkung von PARP 1 untersucht. Dabei zeigte diese Verbindung jedoch nur schwache Wirkung (bei 10 µM 38 % Hemmung).

Es wurde nun überraschenderweise gefunden, daß die erfindungsgemäßen Verbindungen nicht nur auch PARP-Enzyme hemmen, sondern deutlich wirksamer sind (siehe Tabelle).

| Bsp. | PARP1 K_{I}/µM |
|---|---|
| 1 | 0,62 |
| 4 | 0,19 |
| 11 | 1,80 |
| 16 | 0,78 |
| 17 | 0,74 |
| 18 | 0,69 |

Die substituierten Phthalazin-Derivate der allgemeinen Formeln I stellen Inhibitoren der Poly(ADP-ribose)polymerase (PARP) bzw. wie es auch genannt wird Poly(ADP-ribose)synthase (PARS) dar und können somit zur Behandlung und Prophylaxe von Krankheiten, die mit einer erhöhten Enzymaktivität dieser Enzyme verbunden sind, dienen.

Die Verbindungen der Formeln I können zur Herstellung von Arzneimitteln zur Behandlung von Schädigungen nach Ischämien und zur Prophylaxe bei erwarteten Ischämien verschiedener Organe eingesetzt werden.

Die vorliegenden Phthalazin-Derivate der allgemeinen Formel I können danach zur Behandlung und Prophylaxe von neurodegenerativen Krankheiten, die nach Ischämie, Trauma (Schädel-Hirntrauma), Massenblutungen, Subarachnoidal-Blutungen und Stroke auftreten, und von neurodegenerativen Krankheiten wie multipler Infarkt-Dementia, Alzheimer Krankheit, Huntington Krankheit und von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen, und weiterhin zur Behandlung und Prophylaxe von Schädigungen des Herzens nach cardialen Ischämien und Schädigungen der Nieren nach renalen Ischämien, zum Beispiel der akuten Niereninsuffizienz, des akuten Nierenversagens oder von Schädigungen, die während und nach einer Nierentransplantation auftreten, dienen. Weiterhin können die Verbindungen der allgemeinen Formeln I zur Behandlung des akuten Myocardinfarkts und Schädigungen, die während und nach dessen medikamentöser Lyse auftreten (zum Beispiel mit TPA, Reteplase, Streptokinase oder mechanisch mit einem Laser oder Rotablator) und von Mikroinfarkten während und nach Herzklappenersatz, Aneurysmenresektionen und Herztransplantationen dienen. Ebenfalls können die vorliegenden Phthalazine I zur Behandlung einer Revascularisation kritisch verengter Koronaraterien, zum Beispiel bei der PCTA und Bypass-Operationen, und kritisch verengter peripherer Arterien, zum Bespiel Beinarterien, dienen. Zudem können die Phthalazine I bei der Chemotherapie von Tumoren und deren Metastasierung nützlich sein und zur Behandlung von Entzündungen und rheumatischen Erkrankungen, wie z.B. rheumatischer Arthritis und auch zur Behandlung von Diabetes mellitus dienen.

Die erfindungsgemäßen Arzneimittelzubereitungen enthalten neben den üblichen Arneimittel-hilfstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-% enthalten.

Bei der inneren Anwendung werden die Präperationen in Einzeldosen verabreicht. In einer Einzeldosis werden pro kg Körpergewicht 0,1 bis 100 mg gegeben. Die Zubereitung können täglich in einer oder mehreren Dosierungen je nach Art und Schwere der Erkrankungen verabreicht werden.

Entsprechend der gewünschten Applikationsart enthalten die erfindungsgemäßen Arznei-mittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes Hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wikstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verbreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiele

### Beispiel 1

### 4(N(4-Hydroxyphenyl)aminomethyl)-2H-phthalazin-1-on

¹H-NMR (D₆-DMSO): δ = 4,4 (2H), 5,5 (1H), 6,55 (4H), 7,75-8,3 (4H), 8,5 (1H) und ca. 12,5 (1H) ppm.

### Beispiel 2

### 4(N(4-N,N-Dimethylsulfamoyl)phenyl-aminomethyl)-2H-phthalazin-1-on

¹H-NMR (D₆-DMSO): δ = 2,5 (6H), 4,7 (2H), 6.8 (2H), 7,2 (1H), 7,5 (2H), 7,75-8,3 (4H), und ca. 12,5 (1H) ppm.

### Beispiel 3

### 4(N(4-Chlorphenyl)aminomethyl)-2H-phthalazin-1-on

¹H-NMR (D₆-DMSO): δ = 4,6 (2H), 6,4 (1H), 6,75 (2H), 7,1 (2H), 7,9-8,4 (4H) und ca. 12,5 (breit) ppm.

### Beispiel 4

### 4(N-Phenyl)-aminomethyl-2H-phthalazin-1-on

¹H-NMR (D₆-DMSO): δ = 4,6 (2H), 6,3 (1H), 6,2 (1H), 6,8 (2H), 7,1 (2H), 7,9-8,5 (4H) und ca. 12,5 (breit) ppm.

### Beispiel 5

### 4(N(3-Trifluormethyl-phenyl)aminomethyl)-2H-phthalazin-1-on

¹H-NMR (D₆-DMSO) : δ = 4,6 (2H), 6,7 (1H), 6,8 (1), 7,0 (2H), 7,3 (1H), 7,9-8,4 (4H) und ca. 12,5 (breit) ppm.

### Beispiel 6

### 4(N(2-Cyanophenyl)aminomethyl)-2H-phthalazin-1-on

¹H-NMR (D₆-DMSO): δ = 4,8 (2H), 6,7 (2H), 7,1 (1H), 7,5-7,8 (2H), 7,95 (1H), 8,1 (1H), 8,4 (2H) und 12,5 (1H) ppm.

### Beispiel 7

### 4(N(4-Methoxyphenyl)aminomethyl)-2H-phthalazin-1-on

¹H-NMR (D₆-DMSO) : δ = 3,7 (3H), 4,5 (2H), 5,8 (1H), 6,7 (4H), 7,9-8,3 (4H) und ca. 12,5 (breit) ppm.

### Beispiel 8

### 4(N(2,4-Dichlorphenyl)aminomethyl-2H-phthalazin-1-on

¹H-NMR (D₆-DMSO): δ = 4,8 (2H), 6,3 (1H), 7,0 (1H), 7,2 (1H), 7,4 (1H), 7,9 (1H), 8,0 (1H), 8,3 (2H) und ca. 12,5 (breit) ppm.

### Beispiel 9

### 4(N(4-Nitrophenyl)aminomethyl)-2H-phthalazin-1-on

¹H-NMR (D₆-DMSO): δ = 4,8 (2H), 6,8 (2H), 7,8-8,4 (7H) und ca. 12,5 (breit) ppm.

### Beispiel 10

### 4-(N(3-Methylmercapto-phenyl)aminomethyl)-2H-phthalazin-1-on

¹H-NMR (D₆-DMSO): δ = 2,4 (3H), 4,6 (2H), 6,3 (1H), 6,4-6,7 (3H), 7,0 (1H), 7,9-8,4 (4H) und ca. 12,5 (breit) ppm.

### Beispiel 11

### 4(N(2,4-Difluor-phenyl)aminomethyl)-2H-phthalazin-1-on

¹H-NMR (D₆-DMSO): δ = 4,7 (2H), 6,0 (1H), 7,0 (2H), 7,1 (1H), 7,9 (1H), 8,0 (1H), 8,3 (2H) und ca. 12,5 (breit) ppm.

### Beispiel 12

### 4(N(4-Phenoxy-phenyl)aminomethyl)-2H-phthalazin-1-on

¹H-MMR (D₆-DMSO): δ = 4,6 (2H), 6,2 (1H), 6,8 (2H), 6,9 (3H), 7,1 (1H), 7,4 (2H), 8,0-8,5 (4H) und ca. 12,5 (1H) ppm.

### Beispiel 13

### 4(N(4-Trifluormethoxy-phenyl)aminomethyl)-2H-phthalazin-1-on

¹H-NMR (D₆-DMSO): δ = 4,6 (2H), 6,5 (1H), 6,75 (2H), 7,1 1 (2H), 7,8-8,4 (4H) und ca. 12,5 (1H) ppm.

### Beispiel 14

### 4(N(4-Trifluormethyl-phenyl)aminomethyl-2H-phthalazin-1-on

¹H-NMR (D₆-DMSO): δ = 4,7 (2H), 6,8 (2H), 6,95 (1H), 7,4 (2H), 7,8-8,4 (4H) und 12,5 (breit) ppm.

### Beispiel 15

### 4(N-Methyl-N-phenyl-aminomethyl)-2H-phthalazin-1-on x HCl

¹H-NMR (D₆-DMSO): δ = 2,7 (1,5H), 3,0 (1,5H), 3,8 (0,5H), 4,0 (0,5H), 4,5 (1H), 4,9 (1H), 6,5-8,3 (9H) und ca. 12,5 (breit) ppm.

### Beispiel 16

### 4(S(4-Chlorphenyl)mercaptomethyl)-2H-phthalazin-1-on

¹H-NMR (D₆-DMSO): δ = 4,6 (2H), 7,4 (4H), 7,9-8,5 (4H) und ca. 12,5 (breit) ppm.

### Beispiel 17

### 4(S(1-Methyl-imidazol-2-yl)mercaptomethyl)-2H-phthalazin-1-on

### Beispiel 18

### 4(N(5-Methylmercapto-1,3,4-triazol-2-yl)aminomethyl)-2H-phthalazin-1-on

¹H-NMR (D₆-DMSO): δ = 2,4 (3H), 4,7 (2H), 7,1 (1H), 7,8-8,3 (4H) und ca. 12,5 (breit) ppm.

### Beispiel 19

### 4(S(2-Pyridyl)-mercaptomethyl)-2H-phthalazin-1-on

¹H-NMR (D₆-DMSO): δ = 4,8 (2H), 7,2 (1H), 7,4 (1H), 7,7 (1H), 7,9 (1H), 8,0 (1H), 8.2 (1H), 8,5 (1H) und ca. 12,5 (1H) ppm.

## Patentansprüche

1. Verwendung von Verbindungen der Formel 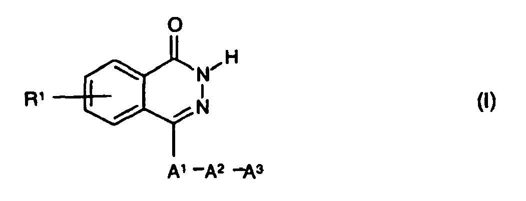 worin
R¹ Wasserstoff, Chlor, Fluor, Brom, Jod, verzweigtes und unverzweigtes C₁-C₆-Alkyl, OH, Nitro, CF₃, CN, NR¹¹R¹², NH-CO-R¹³, O-C₁-C₄-Alkyl, wobei R¹¹und R¹² unabhängig voneinander Wasserstoff oder C₁-C₄-Alkyl bedeuten und R¹³ Wasserstoff, C₁-C₄-Alkyl, C₁-C₄-Alkyl-Phenyl oder Phenyl bedeutet, und
A¹ einen geradkettigen oder verzweigten C₀-C₆-Alkylrest und
A² NR², NR²-C₁-C₆-Alkyl- und S und
R² Wasserstoff und C₁-C₆-Alkyl und
A³ einen aromatischen oder heteroaromatischen ein oder zweigliedrigen Ring mit je 5 oder 6 Ringatomen und bis zu 3 Heteroatomen, ausgewählt aus N, O, S, die noch mit R⁴ und einem oder zwei R³ substituiert sein können, wobei R³ Wasserstoff, Chlor, Fluor, Brom, Jod, verzweigtes und unverzweigtes C₁-C₆-Alkyl, OH, Nitro, CF₃, CN, NR¹¹R¹², ,SO₂NR¹¹R¹², SO₂-C₁-C₄-Alkyl, S-C₁-C₄-Alkyl, O-Ph, O-CF₃, NH-CO-R¹³, O-C₁-C₄-Alkyl, wobei R¹¹ und R¹² unabhängig voneinander Wasserstoff oder C₁-C₄-Alkyl bedeuten und R¹³ Wasserstoff, C₁-C₄-Alkyl, C₁-C₄-Alkyl-Phenyl oder Phenyl bedeuten kann,
R⁴ Wasserstoff, (X)_{0,1}-C₁-C₄-Alkyl-NR⁴¹R⁴², wobei X = O, S und NR⁴³ und R⁴¹ und R⁴² unabhängig voneinander Wasserstoff, C₁-C₆-Alkyl, C₁-C₄-Alkyl-Phenyl und ein cyclisches Amin von 3 bis 7 Gliedern sein kann und R⁴³ Wasserstoff und C₁-C₄-Alkyl sein kann,
sowie ihre tautomeren Formen, möglichen enantiomeren und diastereomeren Formen, und deren Prodrugs zur Herstellung von Arzneimitteln zur Behandlung von neurodegenerativen Krankheiten und neuronalen Schädigungen.

2. Verwendung nach Anspruch 1 zur Behandlung von solchen neurodegenerativen Krankheiten und neuronalen Schädigungen, die durch Ischämie, Trauma oder Massenblutungen ausgelöst werden.

3. Verwendung nach Anspruch 1 zur Behandlung des Schlaganfalls und des Schädel-Hirntraumas.

4. Verwendung nach Anspruch 1 zur Behandlung der Alzheimerschen Krankheit der Parkinsonschen Krankheit und der Huntington-Krankheit.

5. Verwendung von Verbindungen der Formel I gemäß Anspruch 1 zur Herstellung von Arzneimitteln zur Behandlung oder Prophylaxe von Schädigungen durch Ischämien.

6. Verwendung von Verbindungen der Formel I gemäß Anspruch 1 zur Herstellung von Arzneimitteln zur Behandlung von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen.

7. Verwendung von Verbindungen der Formel I gemäß Anspruch 1 zur Herstellung von Arzneimitteln zur Behandlung von Schädigungen der Nieren nach renalen Ischämien und zur Behandlung während und nach Nierentransplantationen.

8. Verwendung von Verbindungen der Formel I gemäß Anspruch 1 zur Herstellung von Arzneimitteln zur Behandlung von Schädigungen des Herzens nach cardialen Ischämien.

9. Verwendung von Verbindungen der Formel I gemäß Anspruch 1 zur Herstellung von Arzneimitteln zur Behandlung von Mikroinfarkten wie zum Beispiel während und nach Herzklappenersatz, Aneurysmenresektionenen und Herztransplantationen.

10. Verwendung von Verbindungen der Formel I gemäß Anspruch 1 zur Herstellung von Arzneimitteln zur Behandlung bei einer Revasculariation kritischer verengter Koronararterien wie zum Beispiel bei PTCA und Bypass-Operationen oder kritisch verengter peripherer Arterien, insbesondere Beinarterien.

11. Verwendung von Verbindungen der Formel I gemäß Anspruch 1 zur Herstellung von Arzneimitteln zur Behandlung des akuten Myocardinfarktes und von Schädigungen während und nach dessen medikamentöser oder mechanischer Lyse.

12. Verwendung von Verbindungen der Formel I gemäß Anspruch 1 zur Herstellung von Arzneimitteln zur Behandlung von Tumoren und deren Metastasierung.

13. Verwendung von Verbindungen der Formel I gemäß Anspruch 1 zur Herstellung von Arzneimitteln zur Behandlung von Sepsis und des septischen Schocks.

14. Verwendung von Verbindungen der Formel I gemäß Anspruch 1 zur Herstellung von Arzneimitteln zur Behandlung von immunologischen Krankheiten wie Entzündungen und rheumatische Erkrankungen, wie zum Beispiel rheumatoide Arthritis.

15. Verwendung von Verbindungen der Formel I gemäß Anspruch 1 zur Herstellung von Arzneimitteln zur Behandlung von Diabetes mellitus.

## Claims

1. Use of compounds of the formula I 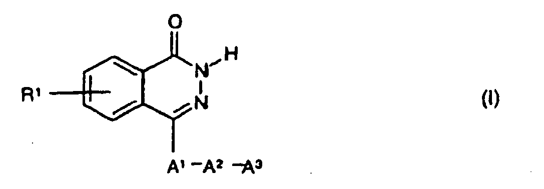 wherein
R¹ is hydrogen, chlorine, fluorine, bromine, iodine, branched and unbranched C₁-C₆-alkyl, OH, nitro, CF₃, CN, NR¹¹R¹², NH-CO-R¹³, O-C₁-C₄-alkyl, wherein R¹¹ and R¹² independently of one another denote hydrogen or C₁-C₄-alkyl and R¹³ denotes hydrogen, C₁-C₄-alkyl, C₁-C₄-alkyl-phenyl or phenyl, and
A¹ is a straight-chain or branched C₀-C₆-alkyl radical and
A² is NR², NR²-C₁-C₆-alkyl and S and
R² is hydrogen and C₁-C₆-alkyl and
A³ is an aromatic or heteroaromatic one- or two-membered ring having in each case 5 or 6 ring atoms and up to 3 heteroatoms selected from N, O, S, which can also be substituted by R⁴ and one or two R³, wherein R³ denotes hydrogen, chlorine, fluorine, bromine, iodine, branched and unbranched C₁-C₆-alkyl, OH, nitro, CF₃, CN, NR ¹¹R¹², SO₂NR¹¹R¹², SO₂-C₁-C₄-alkyl, S-C₁-C₄-alkyl, O-Ph, O-CF₃, NH-CO-R¹³, O-C₁-C₄-alkyl, wherein R¹¹ and R¹² independently of one another denote hydrogen or C₁-C₄-alkyl and R¹³ can denote hydrogen, C₁-C₄-alkyl, C₁-C₄-alkyl-phenyl or phenyl,
R⁴ is hydrogen, (X)_{0,1}-C₁-C₄-alkyl-NR⁴¹R⁴², wherein X = O, S and NR⁴³ and R⁴¹ and R⁴² independently of one another can be hydrogen, C₁-C₆-alkyl, C₁-C₄-alkyl-phenyl and a cyclic amine of 3 to 7 members and R⁴³ can be hydrogen and C₁-C₄-alkyl,
and their tautomeric forms, possible enantiomeric and diastereomeric forms, and prodrugs thereof for the preparation of medicaments for treatment of neurodegenerative diseases and neuronal damage.

2. Use according to claim 1 for treatment of such neurodegenerative diseases and neuronal damage as are caused by ischaemia, trauma or massive bleedings.

3. Use according to claim 1 for treatment of stroke and craniocerebral trauma.

4. Use according to claim 1 for treatment of Alzheimer's disease, Parkinson's disease and Huntington's disease.

5. Use of compounds of the formula I according to claim 1 for the preparation of medicaments for treatment or prophylaxis of damage caused by ischaemias.

6. Use of compounds of the formula I according to claim 1 for the preparation of medicaments for treatment of epilepsies, in particular generalized epileptic seizures, such as, for example, petit mal and tonoclonic seizures, and partial epileptic seizures, such as temporal lobe and complex partial seizures.

7. Use of compounds of the formula I according to claim 1 for the preparation of medicaments for treatment of damage to the kidneys following renal ischaemias and for treatment during and after kidney transplants.

8. Use of compounds of the formula I according to claim 1 for the preparation of medicaments for treatment of damage to the heart following cardiac ischaemias.

9. Use of compounds of the formula I according to claim 1 for the preparation of medicaments for treatment of microinfarctions, such as, for example, during and after heart valve replacement, aneurysm resections and heart transplants.

10. Use of compounds of the formula I according to claim 1 for the preparation of medicaments for treatment in cases of revascularization of critically narrowed coronary arteries, such as, for example, in PTCA and bypass operations, or critically narrowed peripheral arteries, in particular leg arteries.

11. Use of compounds of the formula I according to claim 1 for the preparation of medicaments for treatment of acute myocardial infarction and of damage during and after medicamentous or mechanical lysis thereof.

12. Use of compounds of the formula I according to claim 1 for the preparation of medicaments for treatment of tumours and metastasis thereof.

13. Use of compounds of the formula I according to claim 1 for the preparation of medicaments for treatment of sepsis and septic shock.

14. Use of compounds of the formula I according to claim 1 for the preparation of medicaments for treatment of immunological diseases, such as inflammations, and rheumatic diseases, such as, for example, rheumatoid arthritis.

15. Use of compounds of the formula I according to claim 1 for the preparation of medicaments for treatment of diabetes mellitus.

## Revendications

1. Utilisation de composés de formule 1 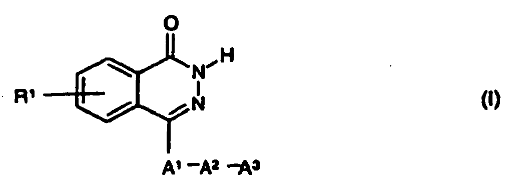 dans laquelle
R¹ représente l'hydrogène, le chlore, le fluor, le brome, l'iode, un reste alkyle en C₁-C₆ linéaire ou ramifié, OH, nitro, CF₃, CN, NR¹¹R¹², NH-CO-R¹³, O-alkyle en C₁-C₄, où R¹¹ et R¹² représentent indépendamment l'un de l'autre l'hydrogène ou un reste alkyle en C₁-C₄, et R¹³ représente l'hydrogène ou un reste alkyle en C₁-C₄, (alkyl en C₁-C₄)-phényle ou phényle, et
A¹ représente un reste alkyle en C₀-C₆ linéaire ou ramifié, et
A² représente NR², NR²-alkyle en C₁-C₆ et S et
R² représente l'hydrogène et un reste alkyle en C₁-C₆, et
A³ représente un système cyclique aromatique ou hétéroaromatique d'un ou deux cycles ayant chacun 5 ou 6 atomes cycliques et jusqu'à 3 hétéroatomes choisis parmi N, O, S, qui peuvent encore être substitués par R⁴ et un ou deux R³, R³ représentant l'hydrogène, le chlore, le fluor, le brome, l'iode, un reste alkyle en C₁-C₆ linéaire ou ramifié, OH, nitro, CF₃, CN, NR¹¹R¹², SO₂NR¹¹R¹², SO₂-alkyle en C₁-C₄, S-alkyle en C₁-C₄, O-Ph, O-CF₃, NH-CO-R¹³, O-alkyle en C₁-C₄, où R¹¹ et R¹² représentent indépendamment l'un de l'autre l'hydrogène ou un reste alkyle en C₁-C₄, et R¹³ peut représenter l'hydrogène ou un reste alkyle en C₁-C₄, (alkyl en C₁-C₄)-phényle ou phényle,
R⁴ représente l'hydrogène, (X)_{0,1}-(alkyl en C₁-C₄)-NR⁴¹R⁴², où X = O, S et NR⁴³ et R⁴¹ et R⁴² peuvent être, indépendamment l'un de l'autre, l'hydrogène, un reste alkyle en C₁-C₆, (alkyl en C₁-C₄)phényle et une amine cyclique de 3 à 7 chaînons, et R⁴³ peut être l'hydrogène et un reste alkyle en C₁-C₄,
et de leurs formes tautomères, de leurs formes énantiomères et diastéréoisomères possibles et de leurs promédicaments pour la préparation de médicaments destinés au traitement de maladies neurodégénératives et de lésions neuronales.

2. Utilisation selon la revendication 1 pour le traitement de maladies neurodégénératives et de lésions neuronales qui sont déclenchées par une ischémie, un traumatisme ou des hémorragies massives.

3. Utilisation selon la revendication 1 pour le traitement de l'attaque d'apoplexie et du traumatisme crâniocérébral.

4. Utilisation selon la revendication 1 pour le traitement de la maladie d'Alzheimer, de la maladie de Parkinson et de la maladie de Huntington.

5. Utilisation de composés de formule 1 selon la revendication 1 pour la préparation de médicaments destinés ou traitement ou à la prophylaxie de lésions dues à des ischémies.

6. Utilisation de composés de formule I selon la revendication 1 pour la préparation de médicaments destinés au traitement d'épilepsies, en particulier de crises épileptiques généralisées comme, par exemple, le petit mal, et de crises tonicocloniques et de crises épileptiques partielles, comme l'épilepsie du lobe temporal, et de crises complexes partielles.

7. Utilisation de composés de formule I selon la revendication 1 pour la préparation de médicaments destinés au traitement de lésions rénales après des ischémies rénales et au traitement pendant et après une transplantation rénale.

8. Utilisation de composés de formule I selon la revendication 1 pour la préparation de médicaments destinés au traitement de lésions cardiaques après des ischémies cardiaques.

9. Utilisation de composés de formule I selon la revendication 1 pour la préparation de médicaments destinés au traitement de microinfarctus comme, par exemple, pendant et après le remplacement d'une valvule cardiaque, des résections d'anévrismes et des transplantations cardiaques.

10. Utilisation de composés de formule I selon la revendication 1 pour la préparation de médicaments destinés au traitement lors d'une revascularisation d'artères coronaires rétrécies critiques comme, par exemple, lors d'opérations d'ACT et de pontages, ou d'artères périphériques rétrécies critiques, en particulier d'artères des jambes.

11. Utilisation de composés de formule 1 selon la revendication 1 pour la préparation de médicaments destinés au traitement de l'infarctus aigu du myocarde et de lésions pendant et après sa lyse médicamenteuse ou mécanique.

12. Utilisation de composés de formule 1 selon la revendication 1 pour la préparation de médicaments destinés au traitement de tumeurs et de leurs métastases.

13. Utilisation de composés de formule I selon la revendication 1 pour la préparation de médicaments destinés au traitement de la septicémie et du choc septique.

14. Utilisation de composés de formule I selon la revendication 1 pour la préparation de médicaments destinés au traitement de maladies immunologiques comme des inflammations et de maladies rhumatismales comme, par exemple, la polyarthrite rhumatoïde.

15. Utilisation de composés de formule I selon la revendication 1 pour la préparation de médicaments destinés au traitement du diabète sucré.
